(19) Europäisches Patentamt
European Patent Office
Office européen des brevets

(11) Veröffentlichungsnummer: **0 240 766**
**A2**

(12) # EUROPÄISCHE PATENTANMELDUNG

(21) Anmeldenummer: **87103583.8**

(51) Int. Cl.[4]: **C07D 239/30**

(22) Anmeldetag: **12.03.87**

(30) Priorität: **22.03.86 DE 3609801**

(43) Veröffentlichungstag der Anmeldung:
**14.10.87 Patentblatt 87/42**

(84) Benannte Vertragsstaaten:
**CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Fauss, Rudolf, Dr.**
**Gerstenkamp 10**
**D-5000 Köln 80(DE)**
Erfinder: **Findeisen, Kurt, Dr.**
**In der Follmühle 10**
**D-5068 Odenthal 2(DE)**
Erfinder: **Schündehütte, Karl Heinz, Prof. Dr.**
**Klief 75**
**D-5090 Leverkusen 3(DE)**
Erfinder: **Thelen, Bernd, Dipl.-Ing.**
**Carl-Duisberg-Strasse 327**
**D-5090 Leverkusen 1(DE)**

(54) **Verfahren zur Herstellung von Tetrachlorpyrimidin.**

(57) Ein einfaches Verfahren zur Herstellung von Tetrachlorpyrimidin besteht darin, 2-Trichlormethyl-4,5,6-trichlorpyrimidin, vorzugsweise in Gegenwart von 0,1 -0,5 Mol Chlor auf ca. 550 - 800°C zu erhitzen.

EP 0 240 766 A2

## Verfahren zur Herstellung von Tetrachlorpyrimidin

Gegenstand der vorliegenden Erfindung ist ein Verfahren zur Herstellung von Tetrachlorpyrimidin (II), dadurch gekennzeichnet, daß 2-Trichlormethyl-4,5,6-trichlorpyrimidin (I) vorzugsweise in Gegenwart von Chlor auf etwa 550 -800°C erhitzt wird.

Das Verfahren kann durch folgende - idealisierte -Gleichungen beschrieben werden

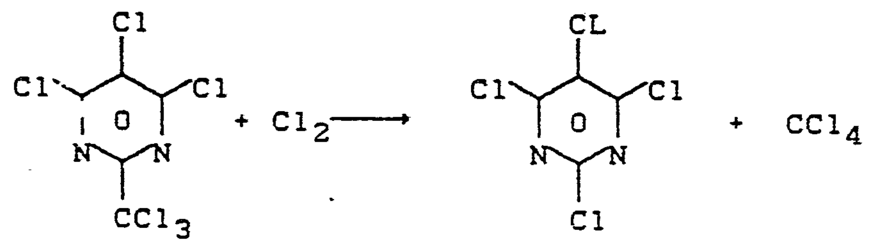

(1)

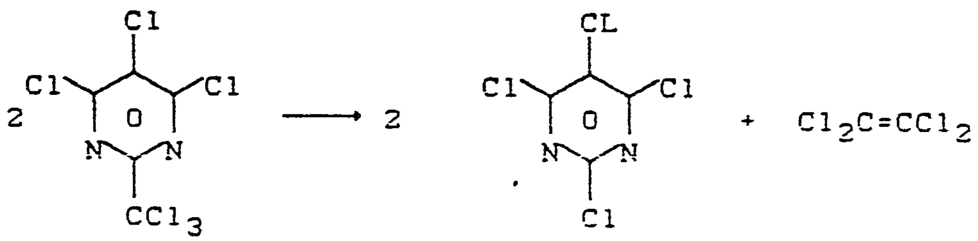

(2)

Je nach der Menge des eingesetzten Chlors verläuft die Reaktion mehr entsprechend der idealisierten Gleichung 1 oder mehr entsprechend der idealisierten Gleichung 2.

Im allgemeinen wird auf 1 Mol (I) 0,1 - 5 Mol Chlor eingesetzt. Ein noch höhrerer Chlorüberschuß wirkt sich nicht nachteilig aus, bringt aber auch keinen Vorteil.

Bevorzugt ist der Einsatz von 0,5 - 2 Mol Chlor je Mol (I) ganz besonders bevorzugt ist ein Verhältnis von 0,9 -1,2 : 1.

Das Arbeiten in Gegenwart von Chlor erweist sich als besonders vorteilhaft. Arbeitet man in Abwesenheit von Chlor, treten auch undestillierbare Rückstände auf.

Der bevorzugte Temperaturbereich ist etwa 650 - 750°C. Die Reaktion erfolgt in der Gasphase.

Die Reaktionstemperatur liegt sinnvollerweise bei 550 -800°C, bevorzugt bei 650 - 750°C. Eine tiefere Temperatur ist auch möglich, dabei sinkt aber die Raum-Zeit-Ausbeute sehr stark ab, so daß ein wirtschaftliches Arbeiten nicht mehr möglich ist. Die Chlorierung verläuft aber nicht in flüssiger Phase, das heißt im Temperturbereich bis ca. 300°C. Es ist auch möglich, bei einer höheren Temperatur als 800°C die Reaktion durchzuführen; jedoch rechtfertigt die dadurch gesteigerte Raum-Zeit-Ausbeute kaum die dabei drastisch steigenden Aufwendungen für Energie und Material.

Die Reaktionszeiten liegen bei ca. 0,1 - 30 sec. Die Reaktion wird zweckmäßigerweise im Strömungsrohr durchgeführt. Hierbei kann (I) entweder flüssig oder in einem Lösungsmittel gelöst zusammen mit dem Chlor in den Reaktor eingegeben werden. Als Lösungsmittel werden bevorzugt die chlorierten Kohlenwasserstoffe Tetrachlorkohlenstoff bzw. Tetrachlorethylen oder deren Mischungen, welche ja auch Reaktionsprodukte sind, eingesetzt.

Die Lösungsmittel sollen keine Wasserstoffatome besitzen, welche bei Reaktionstemperaturen durch Chlor ersetzt werden.

Bevorzugt wird ohne Lösungsmittel gearbeitet. In einer besonders bevorzugten Variante wird das (I) vorverdampft, was bei Normaldruck Temperaturen von 290 - 320°C erfordert, anschließend mit dem entsprechend vorerhitzten Chlorgasstrom gemischt und dann im Strömungsrohr auf Reaktionstemperatur gebracht.

Das Reatktionsrohr, welches aus einem gegenüber den Edukten und Produkten bei Reaktionstemperatur weitgehend inerten Material besteht, kann elektrisch oder durch entsprechende Gas-oder Ölbrenner - respektive durch deren Rauchgase - geheizt werden. Bevorzugt wird die Verwendung eines Quarzrohres in einem elektrisch beheitzten Ofen.

Zur besseren Wärmeübetragung ist es sinnvoll, das Strömungsrohr mit Füllkörpern zu beschicken. Besonders geeignet hierfür sind entsprechend dimensionierte Quarz-Raschigringe.

Die Reaktionszeit läßt sich durch die entsprechende Zupumpgeschwindigkeit der Edukte regeln.

Sie wird zweckmäßigerweise so gewählt, daß gerade alles (I) abreagiert. Eine längere Reaktionszeit ist nicht von Vorteil, bringt aber im allgemeinen außer einer schlechteren Raum-Zeit-Ausbeute auch keinen Nachteil mit sich.

Die optimale Reaktionszeit läßt sich durch einige wenige Vorversuche leicht bestimmen.

Ein elektrisch beheizter Ofen besteht bevorzugt aus mehreren, einzeln regelbaren Temperaturzonen, so daß es gelingt, durch eine hohe Heizleistung am Ofeneingang die Edukte schnell auf Reaktionstemperatur zu bringen.

Die Reaktionsprodukte werden am Ofenausgang möglichst schnell so weit abgekühlt, daß zunächst (II) weitgehend kondensiert. Dies kann durch Außenkühlung oder durch Einspritzen von bereits kondensiertem Produkt geschehen. Im allgemeinen sind hier Temperaturen von unter 200°C bei Normaldruck erforderlich. Die entstandenen Tiefsieder, hauptsächlich Tetrachlorkohlenstoff und/oder Tetrachlorethylen, können sodann in einer zweiten Stufe durch weiteres Abkühlen abgeschieden werden. Selbstverständlich kann diese Kondensation auch in einer Stufe durchgeführt werden. Der dann verbleibende Chlorgasstrom kann einer Wäsche bevorzugt mit Tetrachlorkohlenstoff und/oder Tetrachlorethylen - unterworfen werden, um von rauch-bzw. nebelförmig mitgerissenen Substanzen befreit zu werden.

Er läßt sich anschließend wieder recyclisieren oder wird einer geordneten Vernichtung zugeführt.

Die Aufarbeitung erfolgt im allgemeinen destillativ. Die Ausbeute an (II) liegt im allgemeinen bei über 95 % d. Th.

Der an der Theorie fehlende Teil ist im wesentlichen auf die im Labor nicht völlig zu vermeidenden Aufarbeitungsverluste zurückzuführen.

Die Reaktion läßt sich diskontinuierlich oder kontinuierlich durchführen.

(II) wird beispielsweise als Reaktivkomponente für die Herstellung von Reaktivfarbstoffen verwendet, beispielsweise auch nach Fluorierung zu 2,4,6-Trifluor-5-chlorpyrimidin.

<u>Beispiel 1</u>

Beschreibung der Apparatur

Als Reaktionsrohr dient ein auf einer Länge von 60 cm heizbares Quarzrohr von 3,5 cm Durchmesser, welches mit Quarz-Raschigringen (8 mm Außendurchmesser) gefüllt ist. Ein zentrales Quarzrohr mit 7 mm befindet sich in der Mitte und enthält das Cr-Ni-Thermoelement zur Temperaturmessung und -steuerung. Der Meßpunkt befindet sich ungefähr in der Mitte der Reaktionszone. Das verbleibende Reaktionsvolumen beträgt 320 ml. Das Reaktionsrohr befindet sich in einem elektrisch beheizten Einzonenofen. (I) wird über eine mit Dampf (100°C) beheitzte Kolbenpumpe flüssig von oben auf das senkrecht installierte Reaktionsrohr aufgegeben. Gleichzeitig wird ein konstanter Chlorgasstrom zugeführt. Am Ausgang des Ofens werden die Reaktionsgase in einem mit 65°C heißem Wasser beschickten Intensivkühler abgekühlt und der größte Teil der Produkte als Sumpf aufgefangen. Der überschüssige Chlorgastrom wird dann über einen auf 30°C temperierten aufsteigenden Kühler in einen Waschturm geleitet und anschließend entsorgt. Der Waschturm (80 cm lang, 7 cm Durchmesser, gefüllt mit Glas-Raschigringen) wird mit 5 ltr. Flüssigkeit/h ($CCl_4$) im Kreislauf beaufschlagt.

Durchführung der Reaktion

In 127 min werden 3371 g (I) (96,5 %ig) und 1580 g Chlor bei 700°C durch das Reaktionsrohr geleitet. (Molverhältnis ~ 1:2)

Der Waschturm wird mit 900 ml $CCl_4$ im Kreislauf beaufschlagt. Nach beendeter Reaktion wird 2 x mit 200 ml $CCl_4$ gespült und die Waschturmflüssigkeiten vereinigt.

Anschließend werden Sumpfprodukt und Waschturmflüssigkeit destillativ aufgearbeitet und die einzelnen Fraktionen noch gaschromatographisch untersucht, wobei der genaue Produktgehalt mittels Eichfaktoren festgestellt wird.

Die Hauptfraktion aus dem Sumpfprodukt siedet bei 6 mbar und 92 - 94°C, wiegt 2115 g und enthält 97,3 % (II).

Aus dem Waschturm können noch 147 g 98 %iges (II) erhalten werden.

Wird auch der Gehalt an (II) der Übergangsfraktionen berücksichtigt, so werden 10,71 Mol (II) hergestellt.

Gesamtausbeute: 95,6 % der Theorie.

Beispiel 2

Es wird die Apparatur aus Beispiel 1 verwendet, an Stelle der Quarzraschigringe jedoch Quarzsplitt genommen; das verbleibende Reaktionsvolumen beträgt 200 ml.

In 60 min werden 0,5 Mol (I) als 50 %ige Lösung in Tetrachlorkohlenstoff mit 1,77 Mol Chlor bei 600°C durch das Reaktionsrohr geleitet (Molverhältnis (I) : $Cl_2$ = 1 : 3,54). Die gaschromatographische Untersuchung der Reaktionslösung zeigt, daß 97 % des eingesetzten (I) zu (II) umgesetzt wurden. Arbeitet man mit einem Molverhältnis (I):$Cl_2$ = 1:0,79 erhält man ähnliche Ausbeuten an (II).

**Ansprüche**

1) Verfahren zur Herstellung von Tetrachlorpyrimidin, dadurch gekennzeichnet, daß man 2-Trichlormethyl-4,5,6-trichlorpyrimidin gegebenenfalls in Gegenwart von Chlor auf 550 - 800°C erhitzt.

2) Verfahren nach Anspruch 1, dadurch gekennzeichnet, daß bei ~ 650 - 750°C gearbeitet wird.

3) Verfahren nach Ansprüchen 1 und 2, dadurch gekennzeichnet, daß in Gegenwart von 0,1 - 5 Mol Chlor, vorzugsweise 0,5 - 2 Mol Chlor gearbeitet wird.